# EUROPEAN PATENT APPLICATION

(11) **EP 4 755 873 A1**
(43) Date of publication of application: **10.06.2026**
(21) Application number: 24218110.5
(22) Date of filing: 06.12.2024
(51) Int. Cl.: C07C 323/60, A61K 9/1272, C07D 295/185

(54) **AMINO LIPIDS FOR LIPID-BASED DELIVERY SYSTEMS**

(71) Applicant: Merck Patent GmbH, 64293 Darmstadt (DE); ScreenFect GmbH, 76344 Eggenstein-Leopoldshafen (DE)
(72) Inventor: The designation of the inventor has not yet been filed
(74) Representative: Merck Patent Association

(57) **Abstract**

The invention relates to ionizable cationic amino lipids and lipid-based delivery systems, such as lipid particles comprising ionizable cationic amino lipids of the invention and their use for delivering a payload into a cell.

## Description

### Field of the invention

The invention relates to ionizable cationic amino lipids and lipid-based delivery systems such as lipid particles comprising the same.

### Technical background

mRNA technology has become a powerful approach in the treatment of a wide range of diseases. mRNA molecules are fragile structures that are prone to degradation, particularly by means of enzymatic attack. The safe and functional delivery of nucleic acids to the target tissue is a crucial element for the development of effective preventive and therapeutic applications such as vaccines. Lipid particles, among them lipid nanoparticles (LNPs), are the most clinically advanced delivery vehicle for mRNA and other nucleic acid payloads.

Beyond the established role of LNPs as delivery vehicles in the success of mRNA vaccines against infectious diseases (i.e., SARS-CoV-2 and respiratory syncytial virus), more than 50 different mRNA-LNP drug products are currently in clinical development, with particular focus on infectious diseases, oncology and genetic disorders.

LNPs are typically composed of four lipidic ingredients: An ionizable cationic lipid, a phospholipid and cholesterol (also referred to as helper lipids) and a polymer-lipid conjugate. Each component has a specific role in delivering RNA drugs, with the ionizable cationic lipid being the main performance driver of the LNP delivery vehicle. Owing to the presence of the ionizable lipid, the charge state of the LNP varies as a function of the environmental pH, which in turn affects the safety and performance of the LNP in delivering its payload. As such, the inclusion of an ionizable cationic lipid is key to the success of LNPs.

In WO 2012/167869, an ionizable cationic lipid library is presented consisting of amino lipid structures useful as transfection agents. However, current challenges require further optimization and identification of novel amino lipid structures with enhanced biological performance for *in vivo* delivery of nucleic acid payloads.

The performance of LNPs greatly depends on their fusogenicity, i.e., their ability to merge with the endosomal membrane. LNPs are primarily taken up by cells through endocytosis, after which they are transferred into endosomes. The acidic environment of endosomes causes ionizable lipids of the LNP to become positively charged, increasing their fusogenicity, ultimately leading to the release of the therapeutic payload, such as mRNA, into the cytoplasm. This bypasses potential degradation pathways and increases the likelihood that the therapeutic molecules remain intact and active until they are encountered in the cytoplasm by the molecular machinery of the cell. Optimizing fusogenicity, thus, remains a critical goal in LNP development.

At the same time, safety concerns related to the immunogenicity of LNP remain a critical issue for medical applications. An example of such toxic effect risk is hemolysis, the unintended rupture of red blood cells. While the ability of an LNP to interact with membranes and disrupt them is desirable once inside the endosome, it has the potential of causing damage to cells during circulation in the blood. Excessive hemolysis leads to adverse side effects, compromising the safety profile of LNP-based treatments.

### Objective problem to be solved

To address these challenges, ionizable cationic amino lipid structures are required that improve lipid particle fusogenicity while maintaining a high safety profile, and stable lipid particle physicochemical properties and nucleic acid encapsulation efficiency.

### Summary of the invention

In one aspect, the problem is solved by an amino lipid with the general formula (I): 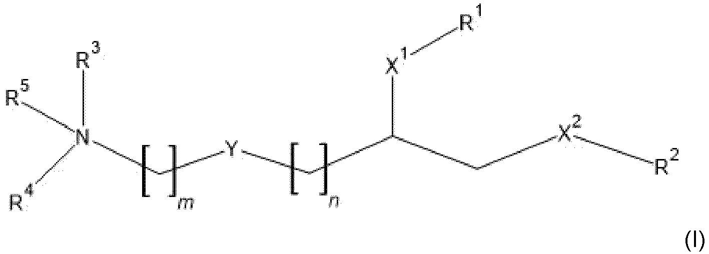
wherein R¹ and R² are the same or different and independently C₁₁ - C₂₄ alkyl or C₁₁ - C₂₄ alkenyl, and at least one of R¹ and R² is C₁₁ - C₂₄ alkenyl,
X¹ and X² are the same or different, either S or S=O or S(=O)₂,

Y is either an amide, ether, ester or a heterocyclic amide of the formula 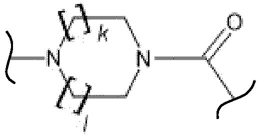
wherein k and I are integers from 0 to 2, R³ and R⁴ are either the same or different and independently hydrogen, C₁ - C₁₂ alkyl, C₁ - C₁₂ alkenyl, or C, - C₁₂ alkynyl, wherein alkyl, alkenyl or alkynyl may be optionally substituted with a C₁ - C₆ hydrocarbyl group, or R³ and R⁴ may join to form an optionally substituted heterocyclic ring of 3 to 10 atoms and 0 to 6 heteroatoms chosen from nitrogen, sulfur and oxygen,
R⁵ is either absent or is hydrogen or C, - C₁₂ alkyl to provide a quaternary ammonium group, and
m is an integer from 0 to 12 and n is an integer from 0 to 12.

In a second aspect, the invention relates to a method for synthesizing an amino lipid according to the invention, comprising the steps of:
a) preparation of an unsaturated alkylthiol by introducing a protective group to an alkyne precursor having one or more triple bonds at the desired locations where the double-bonds are to be located in the amino lipid tail;
b) removal of the protective group;
c) selective reduction of the triple bonds to double bonds, forming an unsaturated intermediate;
d) conversion of the hydroxyl group of the unsaturated intermediate into a thiol;
e) reaction of the intermediate thiol with a methyl ester-derived linker bearing two bromines to form a double-tailed intermediate.
f) amidation of the double-tailed intermediate with an amine to form the final lipid.

In a third aspect, the invention relates to a lipid particle comprising an amino lipid according to the invention.

In a fourth aspect, the invention relates to the use of a lipid particle according to the invention for delivering a payload into a cell.

In a fifth aspect, the invention relates to a pharmaceutical composition comprising an amino lipid or a lipid particle according to the invention.

In a sixth aspect, the invention relates to a vaccine comprising an amino lipid or a lipid particle according to the invention.

### Brief description of the figures

**Fig. 1A** shows the structure of the ionizable cationic amino lipid A1C11.
**Fig. 1B** shows the physicochemical properties of mRNA-loaded LNP formulations containing A1C11 (with varying helper lipid DSPC and DOPE).
**Fig. 1C and 1D** show *in vitro* luciferase activity of HepG2 (C) and C2C12 cells (D) after incubation with LNP formulations (at four different mRNA doses 10 -100 ng). Mean values from triplicates are shown ± standard deviations.
**Fig. 2A** shows structures of tail-group optimized ionizable cationic amino lipids.
**Fig. 2B** shows the physicochemical properties of LNP formulation containing novel tail optimized ionizable cationic amino lipids and the benchmark lipid (MC3).
**Fig. 2C** shows *in vitro* luciferase activity of HepG2 cells after incubation with LNP formulations.
**Fig. 2D** shows the hemolytic effect of LNP formulations containing novel tail optimized ionizable cationic amino lipids and the benchmark lipid (MC3). Mean values from triplicates are shown ± standard deviations.
**Fig. 3A** shows the structure of head-group optimized ionizable cationic amino lipids.
**Fig. 3B** shows the physicochemical properties of mRNA-loaded LNP formulations containing novel head optimized ionizable cationic amino lipids and the benchmark lipid (MC3).
**Fig. 3C** shows the structure of head-group optimized ionizable cationic amino lipids.
**Fig. 3D** shows *in vitro* luciferase activity of HepG2 cells incubated with LNP formulations.
**Fig. 3E** shows the hemolytic effect of LNP formulations. Mean values from triplicates are shown ± standard deviations.
**Fig. 4A** shows the *in vivo* imaging study design.
**Fig. 4B** shows the *ex vivo* imaging study design.
**Fig. 4C** shows whole body *in vivo* bioluminescence signal 6 h post dosing. Mean values from n=3 animals are shown ± standard deviations.
**Fig. 4D** shows *ex vivo* bioluminescence signal 6 h post dosing. Mean values from n=3 animals are shown ± standard deviations.
**Fig. 4E** shows cytokine expression level after 6 h and 24 h post dosing. Mean values from triplicates are shown ± standard deviations.
**Fig. 5** shows luciferase activity as HepG2 cells were treated with LNPs containing mRNA encoding for Luciferase (at four different mRNA doses) for 24 h and the luciferase activity was determined after 24 h. Mean values from triplicates are shown ± standard deviations.
**Fig. 6** shows cell viability as HepG2 cells were treated with LNPs containing mRNA encoding for Luciferase (at four different doses 10 - 100 ng) for 24 h and the cell viability was determined afterwards. Untreated cells were used as control and the cell viability was set to 100%. Mean values from triplicates are shown ± standard deviations.
**Fig. 7** shows luciferase activity as C2C12 cells were treated with LNPs containing mRNA encoding for Luciferase (at four different doses 10 - 100 ng) for 24 h and the luciferase activity was determined afterwards. Mean values from triplicates are shown ± standard deviations.
**Fig. 8** shows cell viability as C2C12 cells were treated with LNPs containing mRNA encoding for Luciferase (at four different doses 10 - 100 ng) for 24 h and the cell viability was determined afterwards. Untreated cells were used as control and the cell viability was set to 100%. Mean values from triplicates are shown ± standard deviations.
**Fig. 9** shows absorption as red blood cells were incubated in PBS at pH = 7.4 (A) (as indication of hemolysis) or in buffer at pH = 5.5 (B) (as indication of membrane fusion capacity) with LNPs for 1 h. The absorption was measured afterwards at 540 nm. Mean values from duplicates are shown ± standard deviations.
**Fig. 10** shows luciferase activity as HepG2 cells were treated with LNPs containing mRNA encoding for Luciferase (at four different doses 10 - 100 ng) for 24 h and the luciferase activity was determined afterwards. Mean values from triplicates are shown ± standard deviations. BM = Benchmark LNP.
**Fig. 11** shows cell viability as HepG2 cells were treated with LNPs containing mRNA encoding for Luciferase (at four different doses 10 - 100 ng) for 24 h and the cell viability was determined afterwards. Untreated cells were used as control and the cell viability was set to 100%. Mean values from triplicates are shown ± standard deviations. BM = Benchmark LNP.
**Fig. 12** shows luciferase activity as C2C12 cells were treated with LNPs containing mRNA encoding for Luciferase (at four different doses 10 - 100 ng) for 24 h and the luciferase activity was determined afterwards. Mean values from triplicates are shown ± standard deviations. BM = Benchmark LNP.
**Fig. 13** shows cell viability as C2C12 cells were treated with LNPs containing mRNA encoding for Luciferase (at four different doses 10 - 100 ng) for 24 h and the cell viability was determined afterwards. Untreated cells were used as control and the cell viability was set to 100%. Mean values from triplicates are shown ± standard deviations. BM = Benchmark LNP.
**Fig. 14** shows cell viability as HepG2 cells were treated with LNPs containing mRNA encoding for Luciferase (at four different doses 10 - 100 ng) for 24 h and the cell viability was determined afterwards. Untreated cells were used as control and the cell viability was set to 100%. Mean values from triplicates are shown ± standard deviations.
**Fig. 15** shows cell viability as HepG2 cells were treated with LNPs containing mRNA encoding for Luciferase (at four different doses 10 - 100 ng) for 24 h and the cell viability was determined afterwards. Untreated cells were used as control and the cell viability was set to 100%. Mean values from triplicates are shown ± standard deviations.
**Fig. 16** shows luciferase activity as C2C12 cells were treated with LNPs containing mRNA encoding for Luciferase (at four different doses 10 - 100 ng) for 24 h and the luciferase activity was determined afterwards. Mean values from triplicates are shown ± standard deviations.
**Fig. 17** shows cell viability as C2C12 cells were treated with LNPs containing mRNA encoding for Luciferase (at four different doses 10 - 100 ng) for 24 h and the cell viability was determined afterwards. Untreated cells were used as control and the cell viability was set to 100%. Mean values from triplicates are shown ± standard deviations.
**Fig. 18** shows absorption as red blood cells were incubated in PBS at pH = 7.4 (A) (as indication of hemolysis), or in buffer at pH = 5.5 (B) (as indication of membrane fusion capacity) with LNPs for 1 h. The absorption was measured afterwards at 540 nm. Mean values from duplicates are shown ± standard deviations.
**Fig. 19** shows absorption as red blood cells were incubated in PBS at pH = 7.4 (A) (as indication of hemolysis), or in buffer at pH = 5.5 (B) (as indication of membrane fusion capacity) with LNPs for 1 h. The absorption was measured afterwards at 540 nm. Mean values from duplicates are shown ± standard deviations.

### Detailed description of the invention

In one aspect, the invention relates to an amino lipid with the general formula (I): 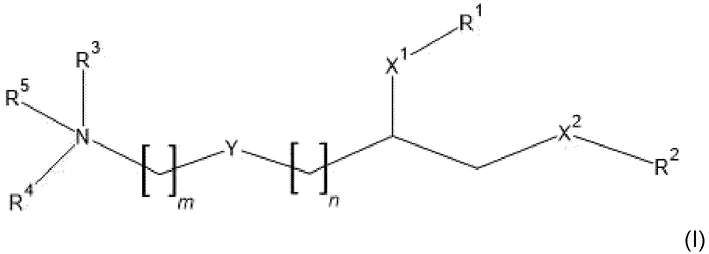
wherein R¹ and R² are the same or different and independently C₁₁- C₂₄ alkyl or C₁₁- C₂₄ alkenyl, and at least one of R¹ and R² is C₁₁-C₂₄ alkenyl,
X¹ and X² are the same or different, either S or S=O or S(=O)₂,
Y is either an amide, ether, ester or a heterocyclic amide of the formula 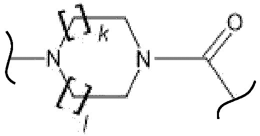
wherein k and I are integers from 0 to 2, R³ and R⁴ are either the same or different and independently hydrogen, C₁-C₁₂ alkyl, C₁-C₁₂ alkenyl, or C₁-C₁₂ alkynyl, wherein alkyl, alkenyl or alkynyl may be optionally substituted with a C₁-C₆ hydrocarbyl group, or R³ and
R⁴ may join to form an optionally substituted heterocyclic ring of 3 to 10 atoms and 0 to 6 heteroatoms chosen from nitrogen, sulfur and oxygen,
R⁵ is either absent or is hydrogen or C₁-C₁₂ alkyl to provide a quaternary ammonium group,
m is an integer from 0 to 12 and n is an integer from 0 to 12.

Amino lipids with longer unsaturated tail moieties at R¹ or R², i.e., at least a C₁₁ alkyl or alkenyl, have been identified by the present inventors as improving lipid particle fusogenicity. In particular, the presence of double bonds in the tail moieties results in an increase in the lipid particle's biological performance and enhanced mRNA delivery.

As used herein, the term "tail moiety" refers to any of the moieties at R¹ and R².

In one embodiment, both R¹ and R² in the amino lipid of the invention are C₁₁-C₂₄ alkenyl. R¹ and R² may be the same or different. Preferably, R¹ and R² are the same. Unsaturated amino lipids outperform previously described amino lipids with regard to delivery efficiency and safety profile. In preferred embodiments, R¹ and R² are C₁₇ or C₁₈ alkenyl.

In a preferred embodiment, at least one C₁₁-C₂₄ alkenyl chain, i.e., at least one of R¹ and R², comprises a double bond located essentially in the middle of the chain, more preferably at position C⁵ to C¹², most preferably at position C⁵, C⁹ or C¹². Herein, C¹ refers to the C-atom closest to X¹ or X², respectively. Double bonds in the middle or near the middle of the tail moiety have a stronger effect on the lipid curvature, leading to a decrease in phase transition temperature and an increase in fluidity.

In another embodiment, at least one alkenyl chain, i.e., at least one of R¹ and R², comprises more than one double bond, e.g., at least two or at least three double bonds. In some embodiments, both tail moieties comprise more than one double bond. In case there are two double bonds in either R¹ or R² or both, the double bonds are preferably at positions C⁹ and C¹². Further decreasing the degree of saturation of the tail moieties of the amino lipid, with 2 or 3 double bonds per chain leads to a significant increase in the fusogenicity of the lipid particle formulation. Significantly enhanced RNA delivery was observed for longer tails comprising multiple double bonds.

The double bonds in the amino lipid of the invention may be either in cis- or trans-configuration independently of each other.

In some embodiments, R⁵ is absent and R³ and R⁴ are either the same or different and independently C₁-C₁₂ alkyl, preferably C₁-C₇ alkyl, more preferably C₁-C₅ alkyl. In the most preferred embodiments, at least one of R³ and R⁴ is C₅ alkyl. The introduction of an alkyl group at these positions, particularly of a pentyl group, leads to a reduction of the pKa and hemolytic potential of the lipid particle.

In other preferred embodiments, at least one, more preferably both, of R³ and R⁴ is methyl.

Preferably, X¹ and X² are both S. Preferably, Y is amide or heterocyclic amide with k and I being 1, most preferably, Y is amide. Preferably, m and n are 2.

In a preferred embodiment, the amino lipid of the invention has one of the following structures:

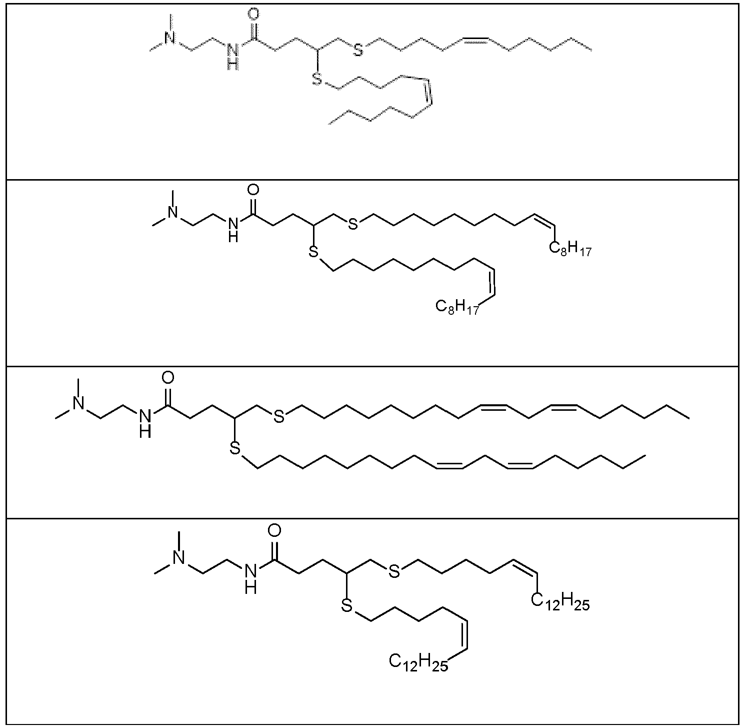

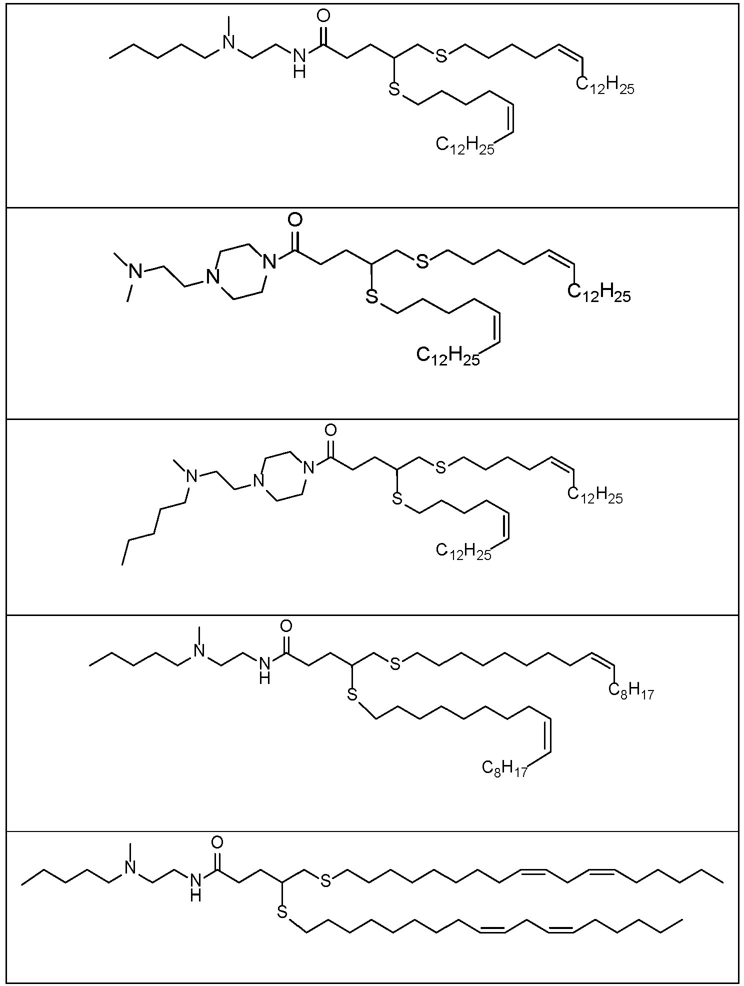

These amino lipids are preferred because their incorporation into lipid particles especially showed improvements in *in vitro* and *in vivo* mRNA delivery efficiency and safety, owing to increased fusogenicity, optimized pKa values and low hemolytic activity. In these terms, the amino lipids outperformed previously identified amino lipids, i.e., up to 100 fold increased mRNA delivery efficiency was observed compared to A1C11 based LNP (see reference Table 1 below), when tested *in vitro,* and up to 140 fold increase, when tested *in vivo*)*,* achieving similar levels of *in vitro* and *in vivo* mRNA delivery efficiency and safety as the market-approved benchmark LNP formulations (i.e., MC3 based LNP, used in Onpattro).

In a further aspect, the invention relates to a method for synthesizing an amino lipid according to the invention, comprising the steps of:
a) preparation of an unsaturated alkylthiol by introducing a protective group to an alkyne precursor having one or more triple bonds at the desired locations where the double-bonds are to be located in the amino lipid tail;
b) removal of the protective group;
c) selective reduction of the triple-bonds to double bonds, forming an unsaturated intermediate;
d) conversion of the hydroxyl group of the unsaturated intermediate into a thiol;
e) reaction of the intermediate thiol with a methyl ester-derived linker bearing two bromines to form a double-tailed intermediate.
f) amidation of the double-tailed intermediate with an amine to form the final lipid.

This modular approach allows for the efficient synthesis of lipid molecules with structural and functional properties beneficial to the lipid particle formulation in terms of fusogenicity and toxicity.

In another aspect, the invention relates to a lipid particle comprising an amino lipid according to the invention.

As used herein, the term "lipid particle" refers to particles having a certain dimension which include one or more specified lipids. In some embodiments, the lipid particle is included in a formulation that can be used to deliver any therapeutic or biologically active molecules to a target site of interest (e.g., cell, tissue, organ, tumor, and the like). Lipid particles according to the invention include lipid nanoparticles (LNPs), lipoplexes (LPXs), liposomes, micelles, lipid-coated nanoparticles, and similar lipid-containing formulations.

In a preferred embodiment, the lipid particle is a lipid nanoparticle (LNP).

As used herein, the term "lipid nanoparticle (LNP)" refers to any nanoscale delivery system composed mainly of lipids, intended to encapsulate and deliver a payload, wherein all three external dimensions of the particle are in the nanoscale, i.e., at least about 1 nm and below about 1000 nm. Preferably, the size of a particle is its diameter.

In one embodiment, a lipid particle according to the invention further comprises a phospholipid, a sterol and a polymer-lipid conjugate.

As used herein, the term "polymer-lipid conjugate" refers to any hybrid structure formed by covalently linking a polymer to a lipid molecule.

In another embodiment, a lipid particle according to the invention further comprises a payload.

As used herein, the term "payload" refers to any therapeutic or biologically active molecules encapsulated within lipid particles for delivery to target cells.

In some embodiments, the payload is a nucleic acid such as a DNA or RNA, preferably a mRNA, siRNA or miRNA, most preferably a mRNA, a protein, preferably an antibody, a drug or an antibody-drug conjugate.

Preferably, the lipid particle according to the invention shows an pKa value between about 6.0 and about 7.0, has low hemolytic activity, low toxicity and and/or improved cell fusogenicity.

The pKa value of the formulation is the pH value at which the LNP becomes protonated. A pKa of between about 6.0 and about 7.0 is considered to be ideal for RNA delivery, since the formulation presents no-to-low charge at physiological pH, which reduces the risk of toxicity in the bloodstream, while in the acidic endosomes, it acquires a higher overall positive charge, leading to the endosomal membrane disruption and release of the payload into the cytoplasm.

In one embodiment, the pKa value is the pKa value as measured using TNS (6-(p-toluidino)-2-naphthalenesulfonic acid sodium salt) assay.

In another preferred embodiment, the lipid particle according to the invention has low toxicity.

As used herein, the term "toxicity" refers to the capacity of lipid particles to cause toxic effects. These may be related to cytotoxicity, hemolytic activity and immunogenicity. So the term "low toxicity" as used herein refers to low cytotoxicity, low hemolytic activity and/or low immunogenicity.

Cellular toxicity (cytotoxicity) is typically associated with the presence of positively charged lipids in the formulation and their effect on the membrane integrity, resulting in cell death.

This toxicity aspect is evaluated by different cell viability assays in vitro (e.g., CellTiter-Glo^{®} determines the number of viable cells in culture based on quantitation of the ATP present, an indicator of metabolically active cells). A healthy cell culture is characterized by more than 80% cell viability. To further evaluate and understand the cell viability (or cytotoxicity) which results from the incubation of the cells with LNP formulations, several controls are used in the assay, such as cells treated with a market-approved LNP benchmark (e.g., D-Lin-MC3-DMA, as used in Patisiran/Onpattro^{®}) (which indicates the acceptable cell viability range), a positive control (i.e., untreated cells) indicating the 100% of viability, and a negative control (i.e., upon incubation of the cells with DMSO) indicating the 0% of viability.

Hemolytic activity refers to the risk of erythrocytes rupturing upon interaction of lipid particles with blood components and is evaluated in vitro by hemolytic assay based on the spectrophotometric measurement of free hemoglobin upon interaction of LNP with erythrocytes at biologically relevant pH. In this context, parenteral formulations with an in vitro hemolysis value lower than 10% are considered to be nonhemolytic. To further evaluate and understand the hemolytic activity of LNP formulations, a control reference (market-approved LNP benchmark such as D-Lin-MC3-DMA, as used in Patisiran/Onpattro^{®}) is used in the assay. Low hemolytic activity thus typically refers to activity lower or equal to the hemolytic activity measured in the case of the interaction of erythrocytes with the control reference.

Immunogenicity induced by lipid particles in vivo is typically associated to the presence of positively charged lipids in lipid particles, because they tend to react with various components of the innate immune system, leading to unwanted immune responses and increased production of different secretory proinflammatory molecules, such as Interleukin 6 (IL-6) or monocyte chemoattractant protein 1 (MCP-1). The immunogenicity of lipid particle formulations upon in vivo injection is measured by ELISA-based assays measuring cytokine induction in animal serum collected at different time points post injection (e.g. using validated singleplex V-plex kits for IL-6 and MCP-1 from Meso Scale Discovery). As absolute cytokine expression levels may vary due to several factors (animal species, age, healthy/disease state), cytokine expression levels are then weighed in relation to untreated animals, wherein an increased cytokine secretion indicates immunogenicity of the lipid particle formulation. To further evaluate and understand the immunogenicity of LNP formulations, a control reference (market-approved LNP benchmark such as D-Lin-MC3-DMA, as used in Patisiran/Onpattro^{®}) is used in the assay, where low immunogenicity typically refers to cytokine expression levels lower or equal to the levels measured in the case of the animals treated with the control reference.

In some embodiments, the lipid particles according to the invention show an increased fusogenicity. In another aspect, the invention relates to the use of a lipid particle according to the invention for delivering a payload into a cell.

In yet another aspect, the invention relates to a pharmaceutical composition comprising an amino lipid according to the invention or a lipid particle according to the invention.

In yet another aspect, the invention relates to a vaccine comprising an amino lipid according to the invention or a lipid particle according to the invention.

### Examples

Ionizable lipids for mRNA delivery, were successfully designed and tested by engineering synthetic unsaturated analogues with more hydrophobic and less exposed protonatable amino head groups. The novel lipids demonstrated improved biological performance, which was particularly evident in both in vitro and in vivo mRNA delivery, showing equal performance compared to current market approved benchmark LNP formulation. The introduction of unsaturated hydrophobic tails and modified ionizable head groups resulted in optimal physicochemical properties, including reduced pKa values compared to previously identified amino lipids, i.e., <7 compared to A1C11 LNP formulations (see reference Table 1 below) and decreased hemolytic activity (at levels equal to market-approved benchmark LNP formulations (i.e., MC3 based LNP, used in Onpattro), while maintaining good nucleic acid complexation and transfection efficiency. The strategic modifications led to effective mRNA delivery with selective organ accumulation, particularly in the liver and spleen. These results indicate that the newly developed lipid structures facilitate efficient cellular uptake and protect mRNA from degradation, addressing the critical issue of translating in vitro efficiency to in vivo performance. Moreover, the novel LNP formulations exhibited low toxicity and reduced hemolytic membrane disruption in vitro.

**Table 1: Structure and working names of referenced amino lipids.**

| **Name** | **Structure** |
|---|---|
| **DLin-MC3-DMA** | 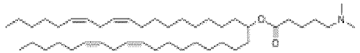 |
| **A1C11** | 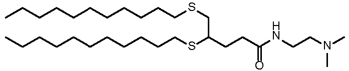 |
| **A1C11_D5** | 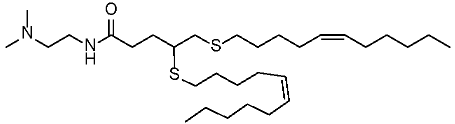 |
| **A1C18_D9** | 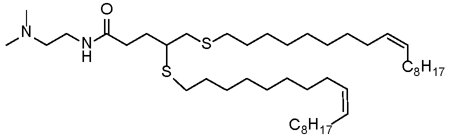 |
| **A1C18_D9_D1 2** | 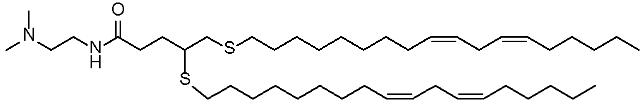 |
| **A1C18_D5** | 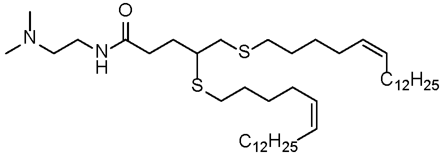 |
| **A1C18** | 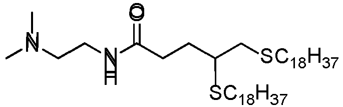 |
| **A2C18_D5** | 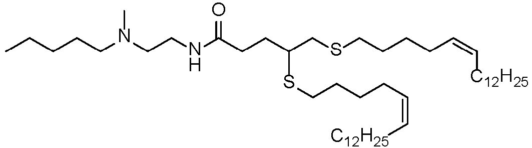 |
| **A3C18_D5** | 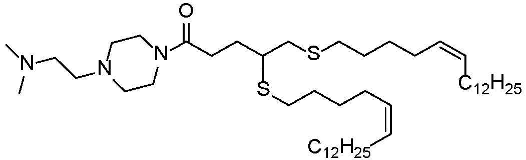 |
| **A4C18_D5** | 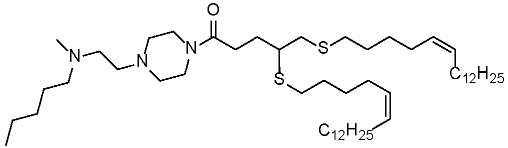 |
| **A2C18_D9** | 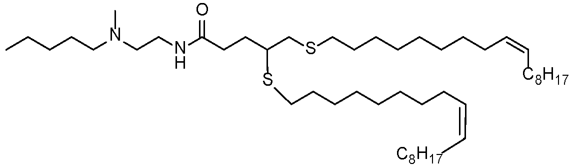 |
| **A2C18_D9_D1 2** | 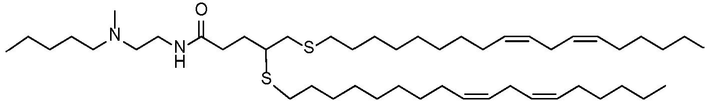 |

### Example 1

### a) Ionizable cationic amino lipids for LNP-mediated mRNA delivery

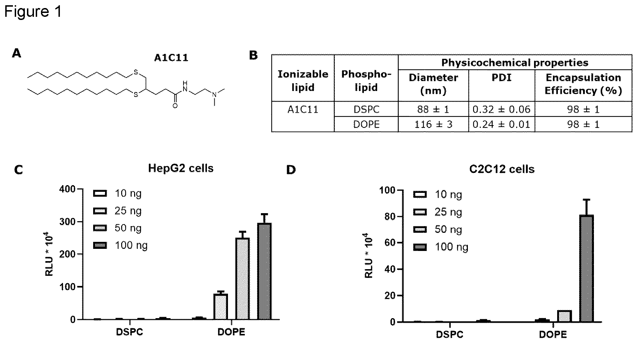

Several top-performing lipids were identified in terms of in vitro delivery of distinct nucleic acid payloads (such as siRNA and plasmid DNA) in "difficult-to-transfect" cell lines. Specifically, with lipid A1C11 (Figure 1A), as transfection reagent (lipoplex formulation), a strong siRNA silencing and a high plasmid DNA transfection rate in vitro was induced.

As a first step, it was decided to test lipid A1C1 1 for its applicability in LNP-mediated mRNA delivery (Figure 1). To this end, LNP were formulated using the same LNP composition as the one found in the marketed Onpattro^{™} LNP formulation. Since the market approval of Onpattro^{™}, its specific LNP composition has been frequently applied in multiple applications, including mRNA delivery for therapeutics and vaccines. MC3, the ionizable lipid found in Onpattro^{™}, was used as the benchmark for the performance evaluation of the novel amino lipid structures tested here.

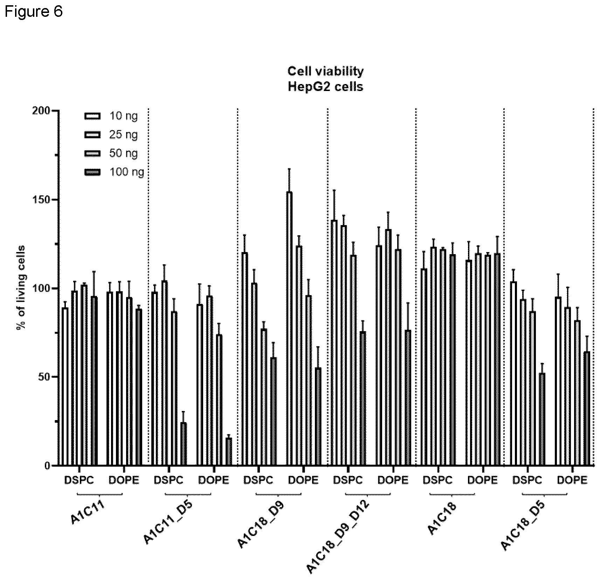

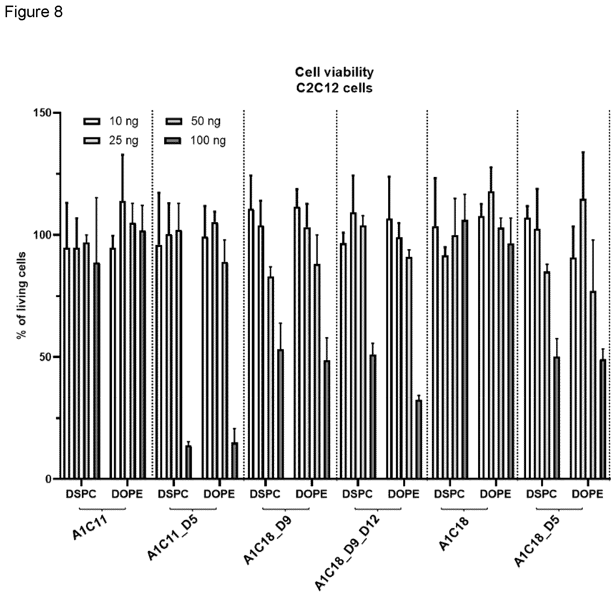

LNP formulations were then prepared with a reporter mRNA (mRNA encoding for firefly luciferase) using the ionizable cationic amino lipid (Figure 1A) and varying the phospholipid type (i.e., DSPC versus DOPE). In line with what has been reported in the literature, the LNP containing DOPE exhibited a larger particle size distribution compared to the ones containing DSPC (Figure 1B). Both LNPs based on A1C11 exhibited high encapsulation efficiency of the mRNA (-98%), while the type of phospholipid had no effect on the apparent pKa of the formulation, which was in both cases >7, indicating that the formulation was positively-charged at physiological pH. Interestingly, when the LNPs were tested in vitro using hepatocyte (HepG2) and muscle (C2C12) cells (Figure 1C, D), the DOPE-containing LNP showed an almost 10-fold higher luciferase activity compared to their DSPC counterparts, while both formulations showed good cell viability at the four doses tested (Figures 6, 8).

It was suspected that a potential reason for this result was associated with the specific contribution of the chosen helper lipid on the membrane fluidity of LNP, which is required for cellular uptake and, more importantly, for endosomal escape. As previously reported, the structural differences of DSPC and DOPE results in differential packing within the LNP, with DOPE adopting a less stable hexagonal phase, while DSPC adopts a more stable lamellar phase. The cone-like geometry of DOPE enables a higher membrane fluidity and fusogenicity, leading to enhanced endosomal release of the RNA cargo. However, DOPE-containing LNPs have been associated with tolerability issues when tested in vivo (due to its enhanced interaction with blood proteins) and, also, with manufacturing and stability challenges.

### b) Optimizing the ionizable lipid tail chemistry to increase LNP fusogenicity

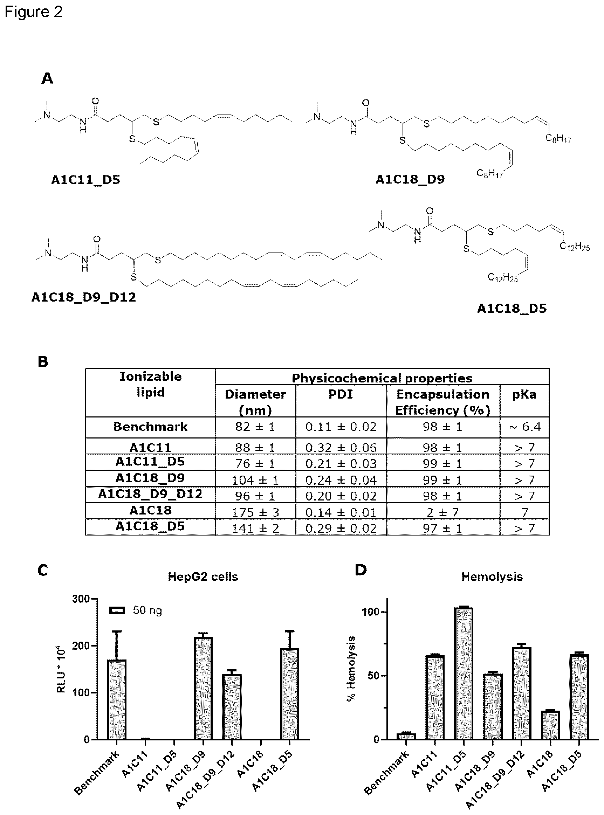

For this reason, it was decided to focus on increasing the fusogenicity of the ionizable cationic lipid itself, rather than relying on helper lipid effects. Specifically, it was decided to design five novel structures, using A1C11 as starting point (Figure 2). In the initial iteration, the effect of double bonds in the alkyl tails of the A1C11 lipid were evaluated, resulting in the lipid A1C11_D5 where 1 set of double bonds was introduced at the C⁵ position for each of the C¹¹ tails.

Four additional lipids with two C¹⁸ chains were designed to mimic the structure of the MC3 benchmark (Figure 2A). At the same time, one or two sets of double bonds per chain were introduced. The position of the double bonds in the new 2×C¹⁸ lipids were placed at the middle (C⁹) of the C¹⁸ chains for lipid A1C18_D9, while for lipid A1C18_D9_D12, the double bonds were positioned at both C⁹ and C¹² on each tail. A1C18 served as the saturated 2xC18 control, in order to distinguish between the effect of the alkyl chains' length and their saturation. Additionally, a control lipid was introduced, A1C18_D5, with one set of double bonds at the C⁵ position of each C¹⁸ chain, allowing for a direct comparison with lipid A1C11_D5, where only the alkyl chain length differed.

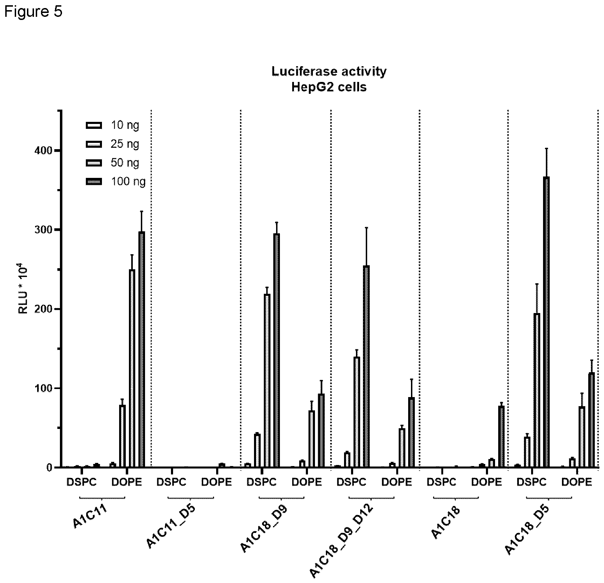

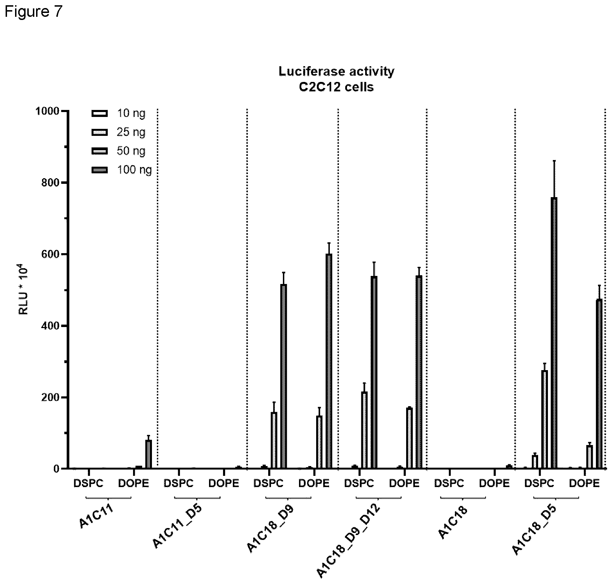

For functional testing, mRNA encoding luciferase was again encapsulated into LNPs formed using these ionizable cationic lipids (Figure 2B), together with DSPC as the helper lipid. Luciferase activity and cell viability tests were performed in HepG2 (Figure 2C) and C2C12 cells (Figures 7, 8). For comparison, LNPs were also formulated with DOPE as the helper lipid (Figure 5-8) and characterized as described above.

All LNPs displayed comparable physicochemical properties (particle size <140 nm, PDI <0.3, and encapsulation efficiency >90%), except for the saturated A1C18-based LNP (Figure 2B). This would be expected due to the more rigid bi-layer of the saturated lipid A1C18.

Of note, significantly enhanced mRNA delivery was observed for the longer C¹⁸ tails harboring one (A1C18_D5 and A1C18_D9) or two (A1C18_D9_D12) sets of double bonds (Figure 2C, Figure 7). Lengthening the chains from C¹¹ to C¹⁸ alone did not show this effect (Figures 5, 7). Interestingly, the helper lipid DOPE did not further enhance the fusogenicity of LNPs composed of unsaturated lipids (HepG2: Figure 2C and Figure 5; C2C12: Figure 7).

### c) Fine tuning the lipid head group to decrease the pKa of the LNP

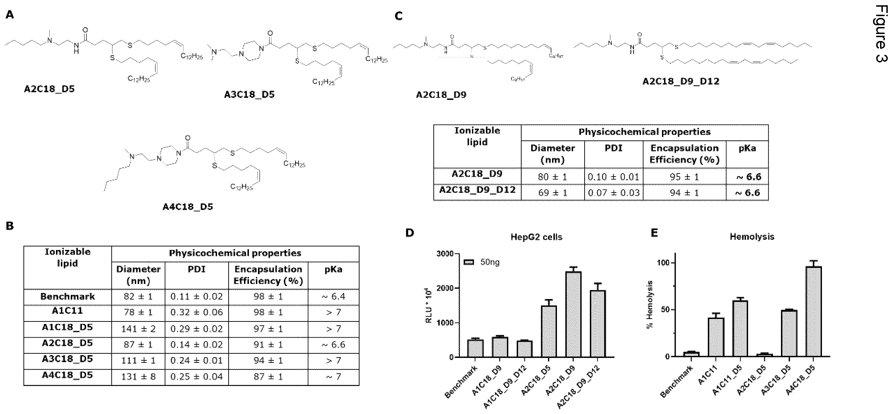

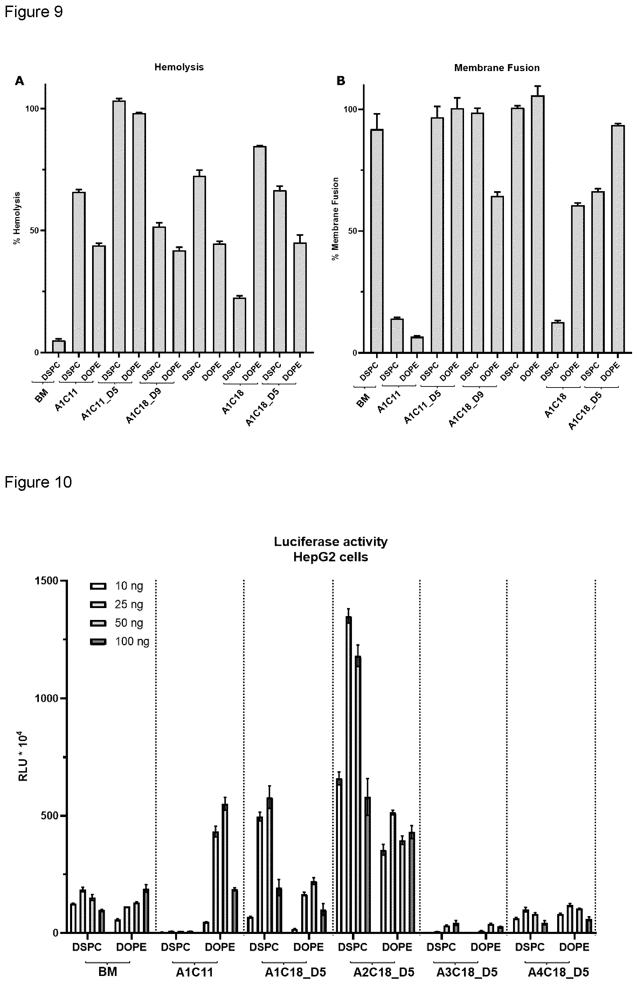

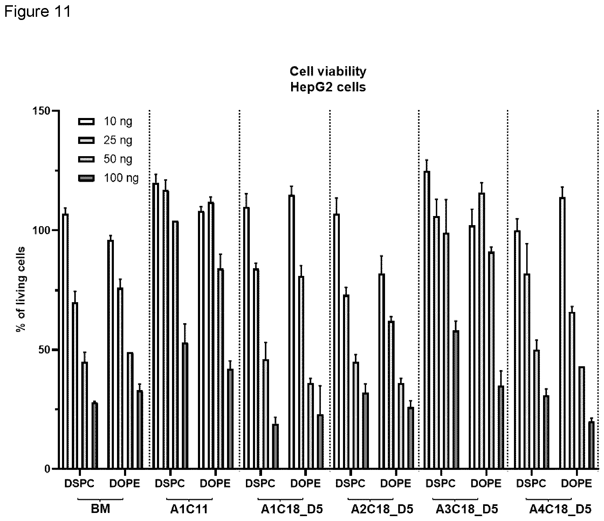

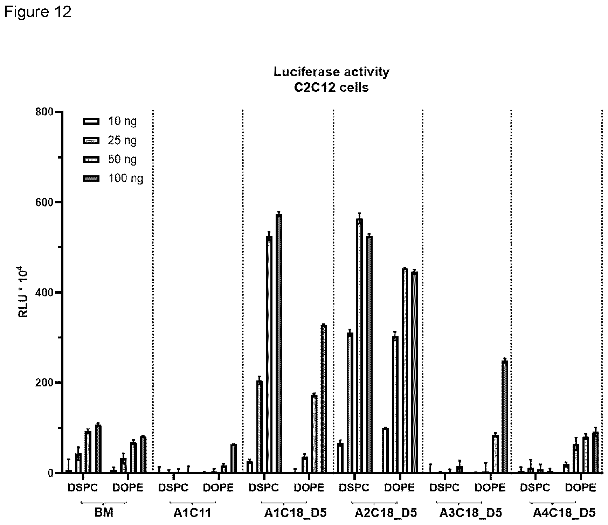

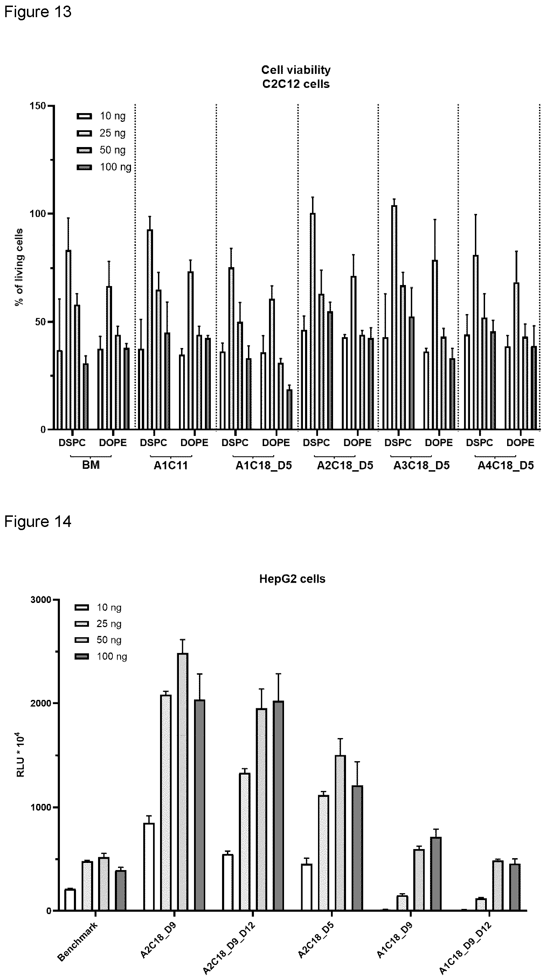

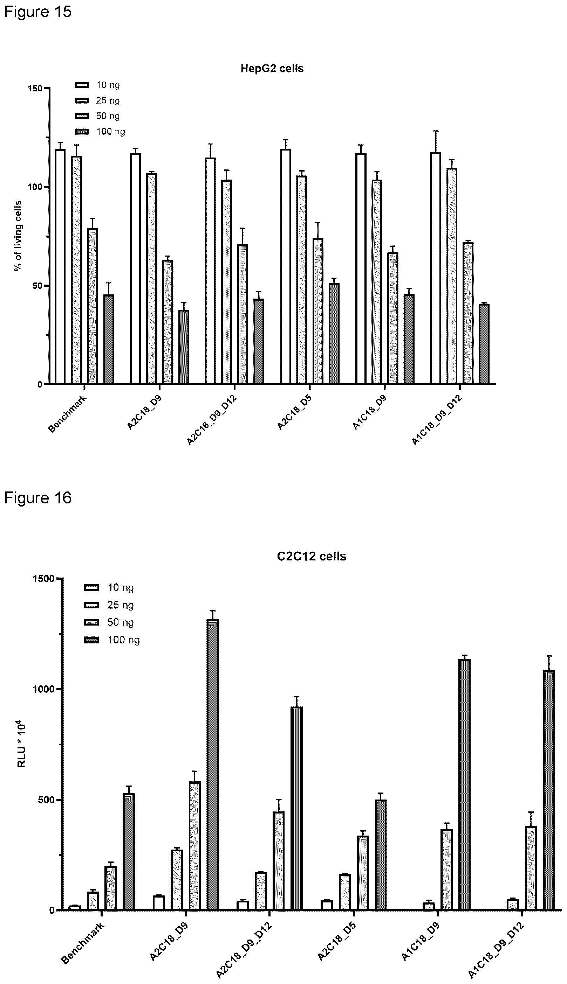

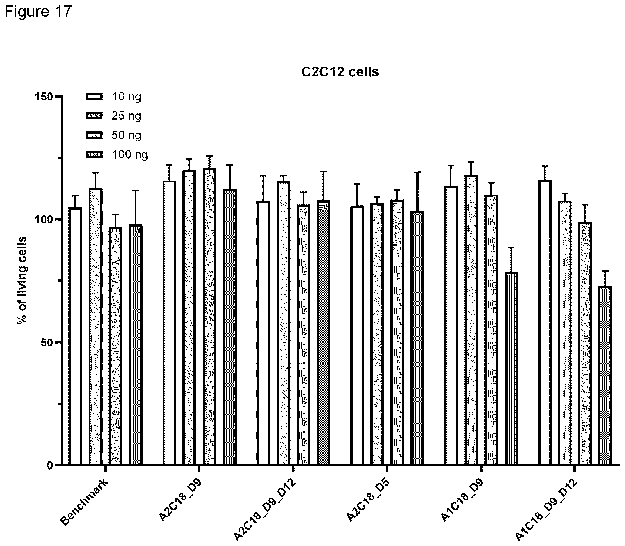

Following these results, the next iterative step was to optimize the pKa of the LNP to the desired value between 6 and 7. Initially, focus was directed to lipid A1C18_D5, modifying the head group of the ionizable lipid as indicated in Figure 3A, generating the lipids A2C18_D5, A3C18_D5, and A4C18_D5. Surprisingly, the introduction of a bulkier, hydrophobic pentyl group (A2) on the head group amine resulted in a significant reduction in the LNP pKa (Figure 3B), while maintaining good particle size distribution and mRNA encapsulation efficiency. With the optimized tails identified above, the lipids A2C18_D9 and A2C18_D9_D12 were additionally synthesized and formulated as described above using DSPC as helper lipid. Remarkably, when tested in vitro in HepG2 (Figure 3D and Figures 10, 14) and C2C12 cells (Figures 12, 16), the LNP's based on lipids A2C18_D5, A2C18_D9 and A2C18_D9_D12 displayed greatly improved delivery efficacy, well above the levels seen for the marketed benchmark composition. Importantly, viability testing in the same cells showed no significant decrease in cell viability for the novel lipids (Figures 11, 13, 15, 17).

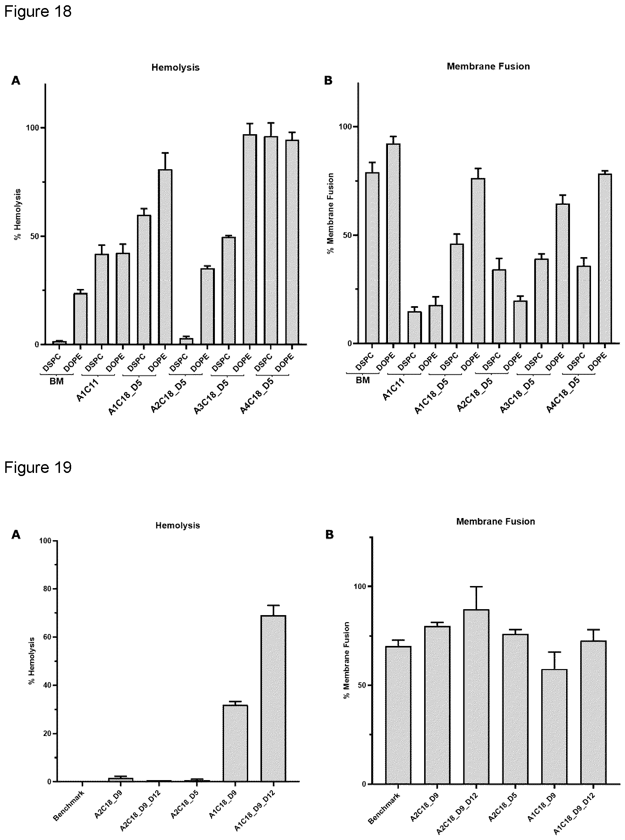

In agreement with the reduced pKa values, LNP containing the novel ionizable lipids A2C18_D5, A2C18_D9 and A2C18_D9_D12 showed greatly reduced hemolysis (Figure 3E) while maintaining high levels of fusogenicity (Figures 18, 19). This may be attributed to the bulkier hydrophobic head group sterically hindering the ability of the tertiary amine to interact with the plasma membrane of blood cells.

### d) Altering the in vivo biodistribution with novel designed LNP formulation

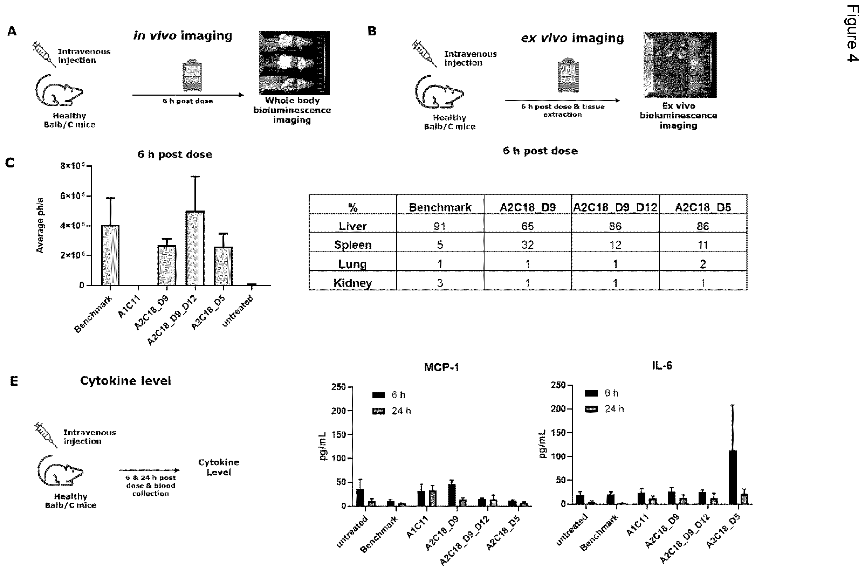

In order to verify the effectiveness of the optimized LNP in vivo, luciferase mRNA-loaded LNP formulations were injected, based on the novel lead lipids A2C18_D5, A2C18_D9 and A2C18_D9_D12, intravenously to Balb/C mice and the distribution of bioluminescence in vivo (whole body bioluminescence) and ex vivo at 6 hours post injection in selected tissues (liver, spleen, kidneys, lungs) studied. As controls, LNP formulations based on the MC3 (benchmark) and our starting point, A1C11, were injected. As can be seen from the results presented in Figure 4, LNP harboring lipids A2C18_D5, A2C18_D9 and A2C18_D9_D12 showed excellent in vivo performance at equal levels to the benchmark formulation, with an impressive more than 200-fold enhancement of protein expression when compared to A1C11 (Figure 4C). Of note, analysis of the ex vivo distribution of bioluminescence after intravenous injection (6 hours post dosing) revealed clear tissue tropism of the novel LNPs to the liver, with the A2C18_D9-based LNPs also showing high spleen localization, resulting in a higher spleen to liver ratio than the marketed LNP benchmark (Figure 4D). This potentially opens the door for extrahepatic targeting. Additionally, animals treated with the novel formulations all showed a similar cytokine expression level (MCP-1 and IL-6) as untreated animals.

### Example 2

### Materials

1,2-Dioleoyl-sn-glycero-3-phosphoethanolamine (DOPE), 1,2-Distearoyl-sn-glycero-3-phosphocholine (DSPC), 1,2-Dimyristoyl-rac-glycero-3-methoxypolyethylene glycol-2000 (DMG-PEG2000), cholesterol, phosphate-buffered saline (PBS) (1x dPBS) and 6-(p-Toluidino)-2-naphthalenesulfonic acid (TNS) were purchased from Sigma-Aldrich (Germany). mRNA encoding for firefly luciferase (CleanCap^{®} FLuc mRNA (5moU)) was acquired from TriLink BioTechnologies (US). Pur-A-Lyzer^{™} Maxi Dialysis Kit (MWCO 12-14 kDa), Amicon Ultra (MWCO 30 kDa) centrifugal units and Millex^{®}-GV filter unit syringe filters (0.22 µm, 13 mm, PVDF) were purchased from MilliporeSigma (Germany). RiboGreen was obtained from ThermoFischer (Germany). All other chemicals and solvents were commercially available and used without further purification.

### Reagents used for the synthesis

Hept-1-yne, potassium thioacetate (KSAc), ammonia (7M), mesyl chloride, N,N-dimethylethane-1,2-diamine, N,N-dimethylaminoethylamine, 1-octadecanethiol, BOC-protected amines (various), pentylchloride, 5% Pd/CaCO₃, Pd/BaSO₄, ethanol, methanol, petroleum ether, dry methanol were purchased from Fisher Scientific (Schwerte, Germany). Dodecyl iodide was purchased from Sigma (Schnelldorf, Germany). N-methyl-1,2-ethylenediamine and trifluoroacetic acid (TFA), dichloromethane (DCM), anhydrous dimethylformamide, diethyl ether, acetic acid, sodium sulfate (Na₂SO₄), celite (for filtration), molecular sieves (for drying solvents) were bought from Carl Roth (Karlsruhe, Germany). Lindlar catalyst (5% Pd/CaCO₃ poisoned with Pd or Pd/BaSO₄) and sodium methylate were obtained from TCI (Eschborn, Germany). Quinoline was received from Merck KGaA (Darmstadt, Germany). Hydrogen gas was purchased from Air Liquide (Ludwigshafen, Germany). 2-(4-lodobutoxy)tetrahydro-2H-pyran (1) and dibromide (25) were obtained from GenoSynth GmbH (Berlin, Germany). Oleyl alcohol, linoleyl alcohol ((9Z,12Z)-octadecadien-1-ol), N1-BOC, N2-methylethane-1,2-diamine, N-methyl-N-pentylamine, BOC-protected compound 30, Hexynol 33, dodecyl iodide, n-butyllithium, triisopropylsilylchloride were purchased from Sigma (Germany).

### Lipid synthesis

### Synthesis of A1C11 D5

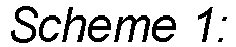

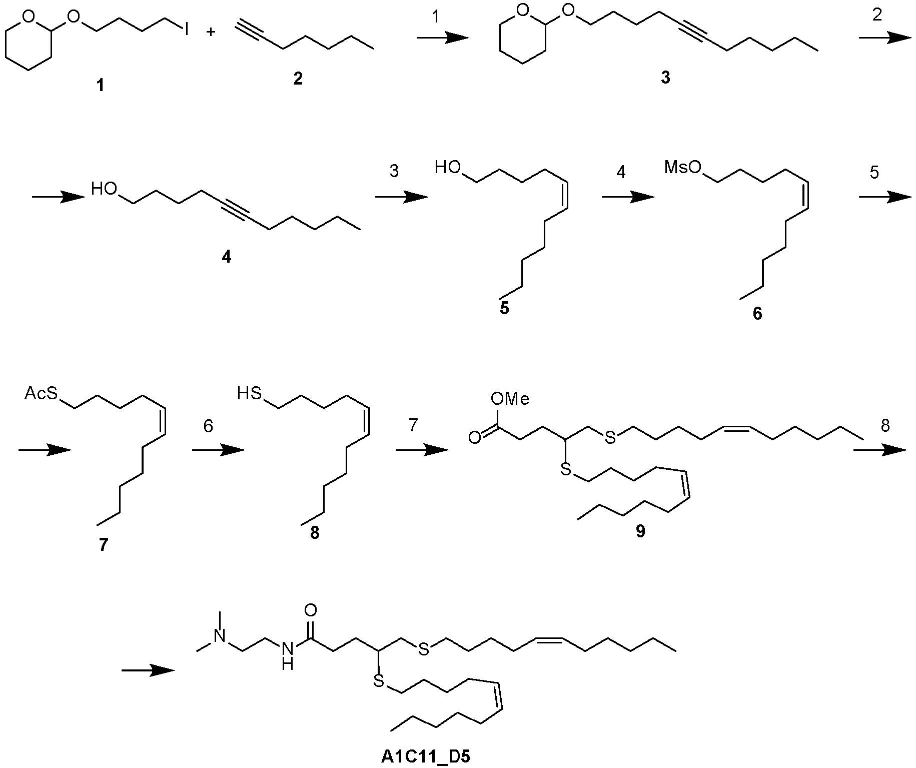

The synthesis began by alkylation of the hept-1-yne (2) with 2-(4-iodobutoxy)tetrahydro-2H-pyran (1) according to Shepherd, J. N. and J. R. Stenzel (2006). "Synthesis of Unsymmetrical Alkynes via the Alkylation of Sodium Acetylides. An Introduction to Synthetic Design for Organic Chemistry Students." Journal of Chemical Education 83(3): 425. This resulted in the formation of the protected alkyne 3. Subsequent acid-catalyzed deprotection of the tetrahydropyranyl (THP) group afforded the corresponding alcohol 4.

Alkyne 4 was then subjected to Lindlar reduction to selectively obtain the alkene 5. The crude alkyne 4 (13.98 g, 83.08 mmol, 1.0 eq.) was added to a 1L three-necked reaction flask, followed by the addition of ethanol (500 mL) as solvent. To the reaction mixture, 5% Pd/CaCO3 (700 mg, poisoned with Pd), Pd/BaSO4 (10% Pd, 700 mg), and quinoline (2 mL, 17.45 mmol, 0.21 eq.) were introduced under a nitrogen atmosphere. The system was purged with hydrogen gas, and the reaction was stirred at room temperature until completion. Upon completion, the reaction mixture was filtered through celite and concentrated under reduced pressure. The crude product was purified by flash chromatography, yielding the alkene 5 as a yellow oil with a 48% yield.

Alcohol 5 was mesylated using mesyl chloride to produce the mesylated compound 6. Subsequently, 6 was reacted with potassium thioacetate (KSAc) to yield AcS-derivative 7. The detailed procedure is as follows: In a 100 mL round-bottomed flask equipped with magnetic stirring, mesylate 6 (11.99 g, 48.3 mmol, 1 eq) was dissolved in anhydrous DMF (50 mL). Potassium thioacetate (11 g, 96 mmol, 2 eq) was added to the reaction mixture in one portion, and the reaction was stirred overnight at room temperature. Upon completion, the mixture was poured into water (500 mL) and extracted with diethyl ether (2 × 200 mL). The organic layers were dried over sodium sulfate (Na₂SO₄), and the solvent was removed under reduced pressure to afford the crude product 7.

The obtained thioester 7 was hydrolyzed to yield thiol 8 according to the procedure: In a 100 mL round-bottomed flask with magnetic stirring, alkenyl thioacetate (11.2 g, 49 mmol, 1 eq) was dissolved in methanol (MeOH) containing 7 M ammonia (28 mL). The reaction was stirred at room temperature overnight. After completion, the reaction mixture was concentrated under reduced pressure and purified by flash chromatography, eluting with neat petroleum ether (PE), to yield thiol 8 as a colorless oil (76% yield).

Compound 9 was synthesized by coupling alkenyl thiol 8 with dibromide 25. In a 500 mL round-bottomed flask with magnetic stirring, sodium methylate (2.17 g, 40.14 mmol, 2.2 eq) was suspended in dry methanol (80 mL, dried over molecular sieves). Alkenyl thiol 8 (7.48 g, 40.14 mmol, 2.2 eq), dissolved in diethyl ether (80 mL, dried over molecular sieves), was added dropwise to the sodium methylate suspension. The mixture was stirred for 30 minutes at room temperature. Dibromide 25 (5 g, 18.24 mmol, 1.0 eq) was then added in one portion, and the reaction was stirred overnight while monitoring progress by TLC. Afterward, acetic acid (1 mL) was added, and the solvent was evaporated under reduced pressure to yield the crude ester 9.

The crude methyl ester 9 was directly subjected to amidation with neat N,N-dimethylaminoethylamine. The ester was dissolved in the amine, and the reaction mixture was heated at 60°C overnight. Upon completion, the solvent was evaporated under reduced pressure, and the crude product was purified by flash chromatography to afford the target lipid A1C11_D5.

### Synthesis of A1C18 D9

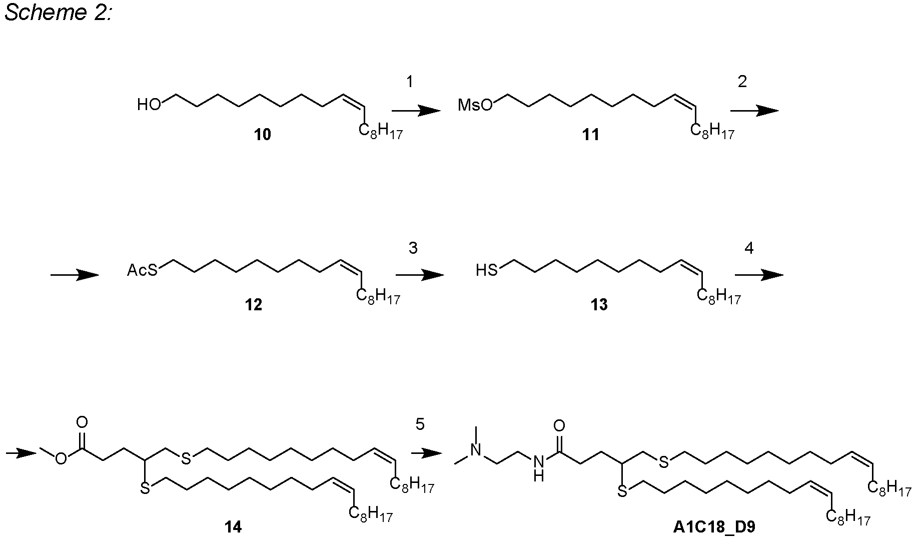

A1C18_D9 was synthesized following a procedure analogous to that used for A1C11_D5, beginning with commercially available oleyl alcohol 10. The alcohol was first mesylated using mesyl chloride, yielding mesylated intermediate 11. This intermediate was then treated with potassium thioacetate (KSAc), resulting in the formation of the AcS-derivative 12. Subsequent hydrolysis of compound 12 produced thiol 13, which was further reacted with dibromide 25. The final step involved direct amidation with dimethylaminoethyl, leading to the target lipid, A1C18_D9.

### Synthesis of A1C18 D9 D12

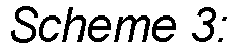

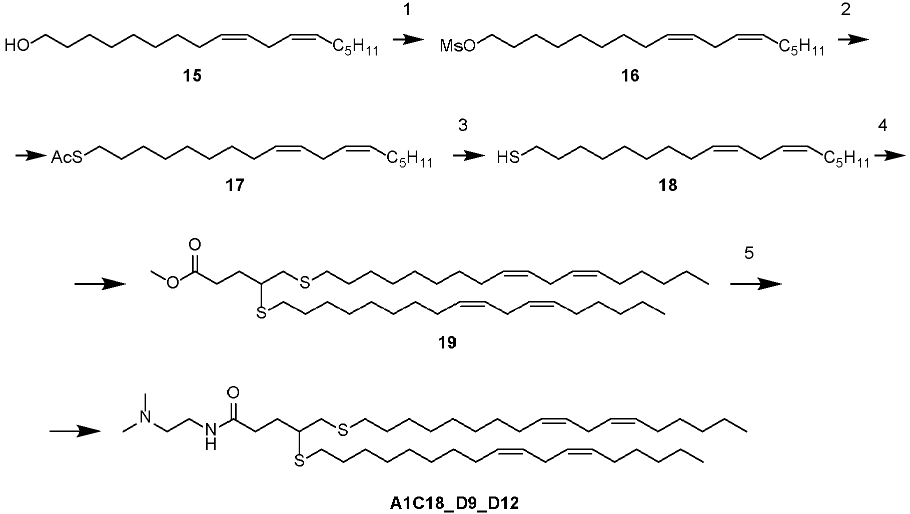

A1C18_D9_D12 was synthesized following the same procedure used for A1C11_D5, starting from linoleyl alcohol 15 ((9Z,12Z)-octadecadien-1-ol) as the initial material.

### Synthesis of A1C18

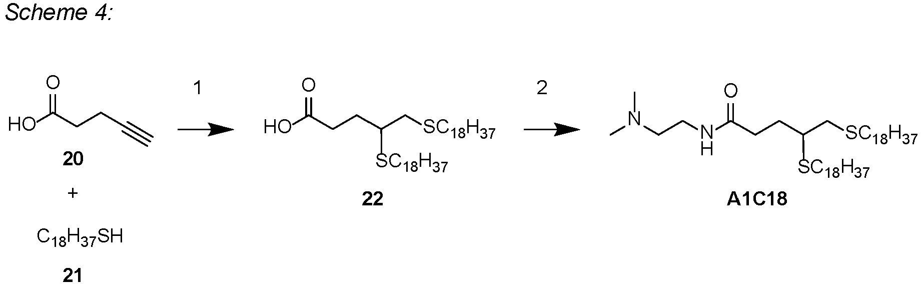

A1C18 was synthesized following a published two-step procedure. In the first step, alkyne 20 was reacted with 1-octadecanethiol through a UV-induced thiol-yne reaction. This was followed by the amidation of the resulting carboxylic acid with the corresponding amine, as described in Linxian Li, David Zahner, Yi Su, Christoph Gruen, Gary Davidson, Pavel A. Levkin, A biomimetic lipid library for gene delivery through thiol-yne click chemistry, Biomaterials, Volume 33, Issue 32, 2012, Pages 8160-8166, ISSN 0142-9612 to give A1C18.

### Synthesis of precursors, dibromide 25, and amines 28 and 32

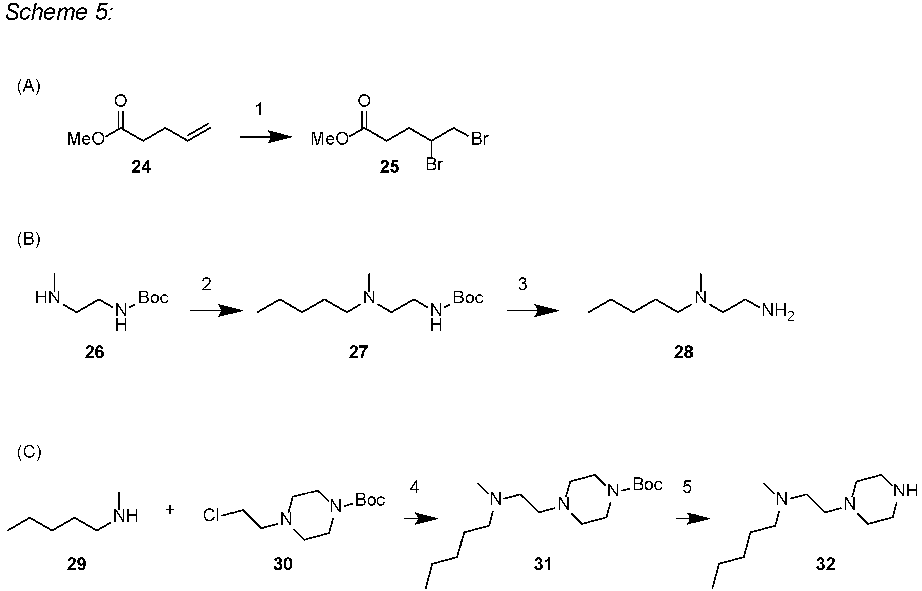

Dibromide 25 was synthesized by addition of brom to alkene 24 following a standard procedure [Organikum, 23. Edition, Wiley-VCH, 2009].

Amine 28 was prepared by alkylating N¹-BOC, N²-methylethane-1,2-diamine 26 with pentylchloride, followed by BOC deprotection using trifluoroacetic acid (TFA) in dichloromethane (DCM), yielding amine 28.

In a similar manner, amine 32 was synthesized by alkylating N-methylpentan-1-amine 29 with the BOC-protected compound 30. Subsequent BOC deprotection yielded the desired amine 32.

### Synthesis of A1C18 D5, A2C18 D5, A3C18 D5 and A4C18 D5

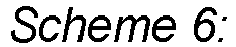

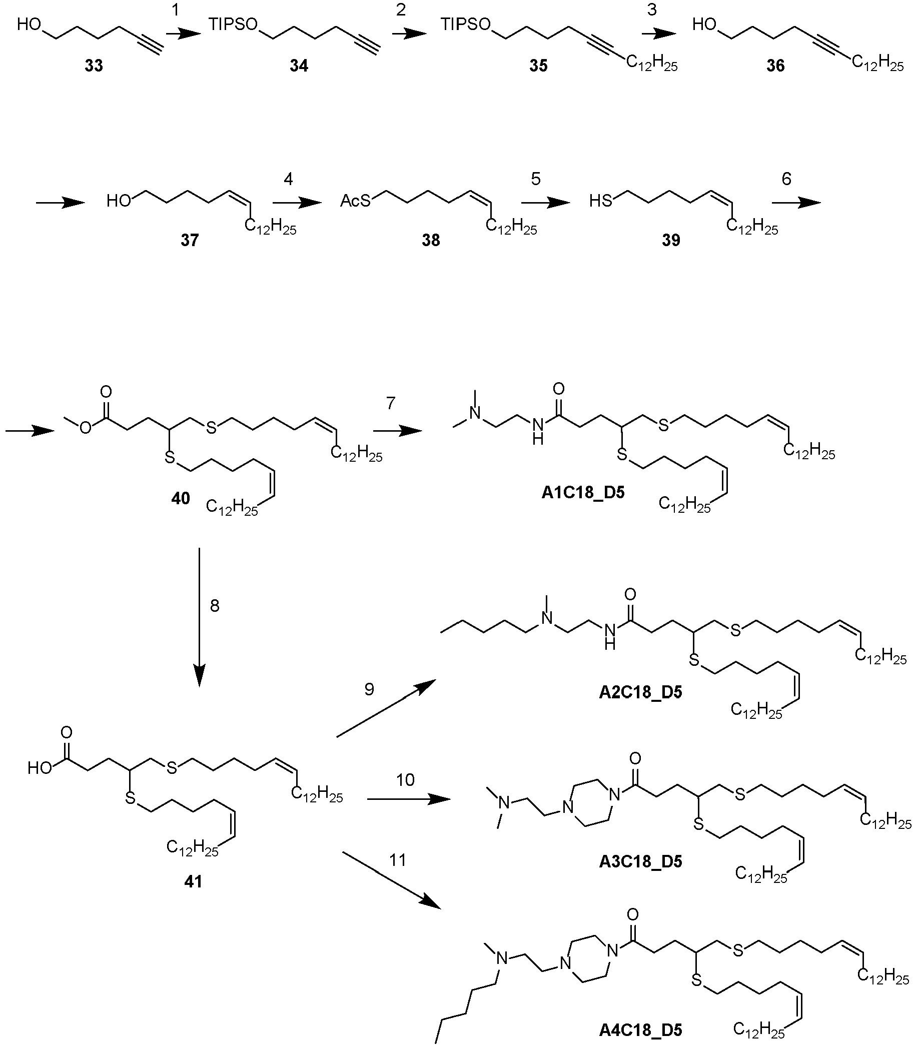

Hexynol 33 was initially protected with a triisopropylsilyl (TIPS) group, followed by alkylation with dodecyl iodide (C₁₂H₂₅I) using n-butyllithium to yield alkyne 35. Afterward, the TIPS group was removed to produce octadec-5-yn-1-ol 36, which was then selectively reduced to (Z)-octadec-5-en-1-ol 37 using a Lindlar catalyst, following the procedure previously described for the synthesis of alkene 5. Octadecenol 37 was converted to (Z)-octadec-5-ene-1-thiol 39 and subsequently transformed into methyl ester 40 bearing two C18_D5 tails, as outlined in the procedure used for compound 9. Methyl ester 40 was hydrolyzed to obtain carboxylic acid 41, which was then subjected to amidation with various amines - 28, N,N-dimethyl-2-(piperazin-1-yl)ethan-1-amine, and amine 32 -to synthesize lipids A2C18_D5, A3C18_D5, and A4C18_D5, respectively. For the synthesis of lipid A1C18_D5, methyl ester 40 was directly amidated with N,N-dimethylethane-1,2-diamine using the same method described earlier for compound A1C11_D5.

### Synthesis of A2C18 D9

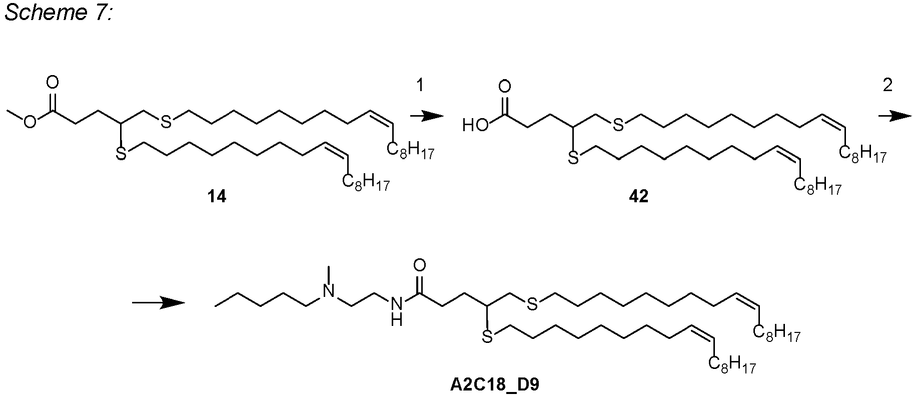

Lipid A2C18_D9 was synthesized from methyl ester 14 by its hydrolysis to the carboxylic acid 42, followed by its amidation using amine 28 to give the final lipid.

### Synthesis of A2C18 D9 D12

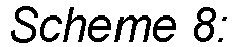

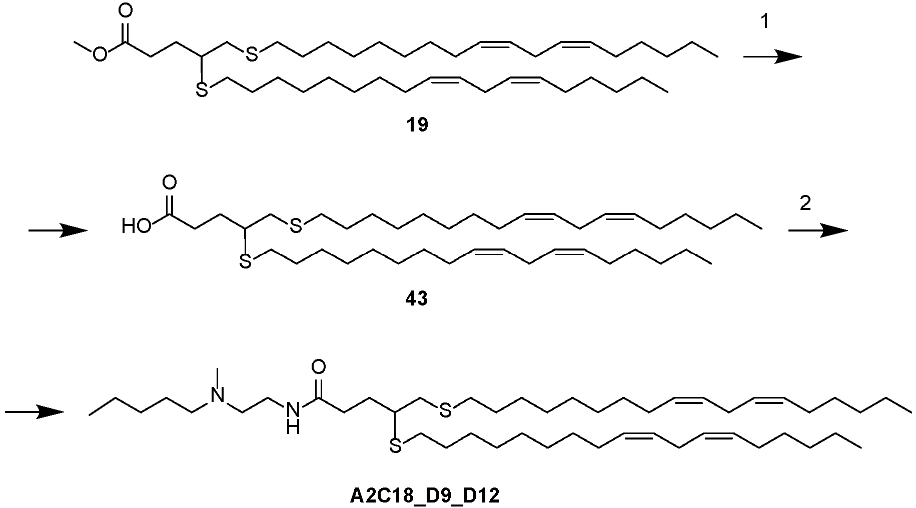

Lipid A2C18_D9_D12 was synthesized from methyl ester 19 by its hydrolysis to the carboxylic acid 43, followed by its amidation using the amine 28 to give the final lipid.

### LNP formulation and characterization

LNP with a lipid composition of 50:38.5:10:1.5 mol% (ionizable lipid:cholesterol:phospholipid:DMG-PEG2000) were prepared by mixing an ethanolic lipid solution with a mRNA aqueous solution in 50 mM citrate buffer at pH 4 at a mRNA concentration of 0.15 mg/mL, using a commercial microfluidic-based mixing device (mRNA:lipid phase flow rate ratio = 3:1, total flow rate = 12 mL/min), to result in mRNA formulated in LNP at a nitrogen-to-phosphate (N/P) ratio of 6. The formulations were dialyzed overnight (at 2-8 °C) against 100-fold volume of 1x dPBS or the appropriate storage matrix buffer. After dialysis, the formulations were up-concentrated to the desired concentration, introduced to the appropriate storage matrix by dilution and finally sterile filtered.

Particle size and polydispersity was determined using a DynaPro^{™} Plate Reader III equipment (Wyatt Technology, US). The mRNA concentration and encapsulation efficiency of the final formulations were determined using the RiboGreen assay and a mRNA standard curve and comparing fluorescence in the presence and absence of Triton X-100, using a Tecan Infinite M200 Pro Multi Mode Microplate Reader (Tecan, US).

### Evaluation of in-situ pKa by TNS assay

For the TNS assay, 20 mM citrate/phosphate buffer series (in 150 mM NaCl), covering a pH range between 4.0 - 8.0 (with increments of 0.2), were prepared. In the wells of a 96-well plate, 10 µL of the LNP solution (0.2 mM total lipid), 88 µL of the each of the buffers above and 2 µL of the TNS solution (0.3 mM stock solution in water) were mixed. The fluorescence of the TNS (ex: 322 nm, em: 431 nm) was measured using a microplate reader. Fluorescence for each formulation at the various pH values was then normalized to the value at pH 4.0. By assuming that minimum fluorescence represents zero charge, and maximum fluorescence represents 100% charge, pKa was estimated by measuring the pH at the point exactly halfway between the values of minimum and maximum charge using Prism10.2.1 (GraphPad, US).

### Hemolysis and membrane fusion assay

Human blood (healthy donors from blood donation bank at Merck KGaA, Darmstadt, Germany) was centrifuged for 5 min at 500 × g. The plasma was aspirated, the isolated human red blood cells were washed twice with 1x dPBS and diluted in either 1x dPBS or citrate buffer saline at pH 5.5 (CBS, 20 mM citrate buffer, 130 mM NaCl) to a 4% vol/vol red blood cell suspension. In a 96-well plate, 40 µL of LNP formulated at a mRNA concentration (0.01 mg/mL) were added to 40 µL of the 4% vol/vol red blood cell suspension in either 1x dPBS or CBS and heated to 37 °C for 1 h. After cooling, the plate was centrifuged at 500 × g and 4 °C for 5 min; the supernatant was transferred into another 96-well assay plate and the absorption was read at 540 nm. Positive and negative controls were carried out with 0.2% Triton-X (100%) and 1x dPBS alone, respectively.

### In vitro transfection

In vitro transfection was performed using HepG2 and C2C12 cells (ATCC, US). HepG2 cells were maintained at 37 °C in a 5% (vol/vol) CO2 atmosphere in Eagle's Minimium Essential Medium) with 10% (vol/vol) fetal bovine serum (FBS), 1x non-essential amino acid (ThermoFisher), 200 mM glutamine and 1x PenStrep (all from Sigma-Aldrich, Germany). C2C12 cells were maintained at 37 °C in a 5% (vol/vol) CO2 atmosphere in Dulbecco's Modified Eagle Medium (high glucose) with 10% (vol/vol) FBS and 1x PenStrep) for a maximum of 20 passages. Cells were passaged 2-3 times per week. Before transfection 10.000 HepG2 or 5.000 C2C12 cells were seeded in white 96-well plates and allowed to attach overnight. The next day, the media was replaced with fresh media containing LNP at a dose of 10, 25, 50 and 100 ng (mRNA dose). Relative firefly luciferase activity and cell viability was assessed ~24 h after LNP addition using ONE-Glo^{™} + Tox Luciferase Reporter and Cell Viability Assay (Promega, Germany).

### In vivo biodistribution and tolerability

Animal studies were carried out in collaboration with the Innovation Campus Berlin (Nuvisan ICB GmbH) and in strict accordance with AAALAC guidelines and the European Directive 2010/63/EU as well as the German Animal Welfare Act (Tierschutzgesetz). Animal experiments were approved by the Institutional Animal Care and Use Committee and competent regional Animal Care and Use Committees according to §15 TierSchG (application number E0155/23; Berlin, Germany).

Eight-week-old female BALB/c mice were purchased from Janvier Labs and were housed in a Specific and Opportunistic Pathogen Free animal facility. Mice were allowed to acclimatize for at least 1 week. Mice had free access to food and water and were exposed to 12-h light/dark cycles. Body weight was determined daily. Severity grade/ well-being of each animal is controlled and documented twice daily.

Groups of six mice each received one intravenous injection (single dose: 0.25 mg/kg of encapsulated mRNA in 10 mL/kg) of each tested LNP formulation per group. At 6 hours post dosing, 3 animals of each group underwent whole body live Bioluminescence imaging (BLI). For BLI, 3 animals per time point from each group received Luciferin (150mg/kg, i.p.) and underwent full body bioluminescence in the BERTHOLD TECHNOLOGIES NightOwl device 10 min later under isofluran narcosis. After whole body BLI, the 3 animals from each group were sacrificed and selected organs (lung, liver, heart, spleen,) were harvested for ex vivo BLI. The rest of 3 animals from each group were sacrificed by heart puncture at 24 hours post dosing, and blood plasma for cytokine level analysis was harvested. Concentrations of cytokines and chemokines in the animal plasma, collected at both 6 and 24 hours post dosing, were quantified using validated singleplex V-plex kits from Meso Scale Discovery (MSD) according to the manufacturer's instruction, for IL-6 and MCP-1 (V-PLEX Mouse MCP-1 Kit and V-PLEX Mouse IL-6 Kit, respectively).

## Claims

1. An amino lipid with the general formula (I): 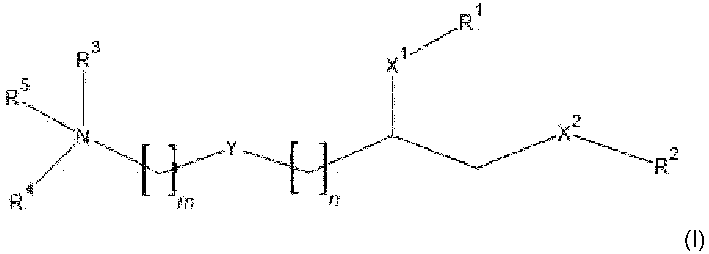
wherein R¹ and R² are the same or different and independently C₁₁- C₂₄ alkyl or C₁₁-C₂₄ alkenyl, and at least one of R¹ and R² is C₁₁-C₂₄ alkenyl,
X¹ and X² are the same or different, either S or S=O or S(=O)₂,
Y is either an amide, ether, ester or a heterocyclic amide of the formula 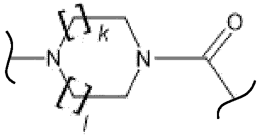
wherein k and I are integers from 0 to 2, R³ and R⁴ are either the same or different and independently hydrogen, C₁-C₁₂ alkyl, C₁-C₁₂ alkenyl, or C₁-C₁₂ alkynyl, wherein alkyl, alkenyl or alkynyl may be optionally substituted with a C₁-C₆ hydrocarbyl group, or R³ and R⁴ may join to form an optionally substituted heterocyclic ring of 3 to 10 atoms and 0 to 6 heteroatoms chosen from nitrogen, sulfur and oxygen,
R⁵ is either absent or is hydrogen or C₁-C₁₂ alkyl to provide a quaternary ammonium group,
m is an integer from 0 to 12 and n is an integer from 0 to 12.

2. The amino lipid of claim 1, wherein both of R¹ and R² are C₁₁-C₂₄ alkenyl, wherein preferably R¹ and R² are the same.

3. The amino lipid according to claim 1 or 2, wherein R⁵ is absent and R³ and R⁴ are either the same or different and independently C₁-C₁₂ alkyl, preferably C₁-C₇ alkyl, most preferably C₁-C₅ alkyl.

4. The amino lipid according to any of claims 1 to 3, wherein at least one C₁₁-C₂₄ alkenyl chain comprises a double bond located essentially in the middle of the chain, preferably at position C⁵ to C¹², most preferably at position C⁵, C⁹ or C¹².

5. The amino lipid according to any of claims 1 to 4, wherein at least one alkenyl chain contains more than one double bond, preferably at positions C⁹ and C¹².

6. The amino lipid of any of claims 1 to 5, having one of the following structures:

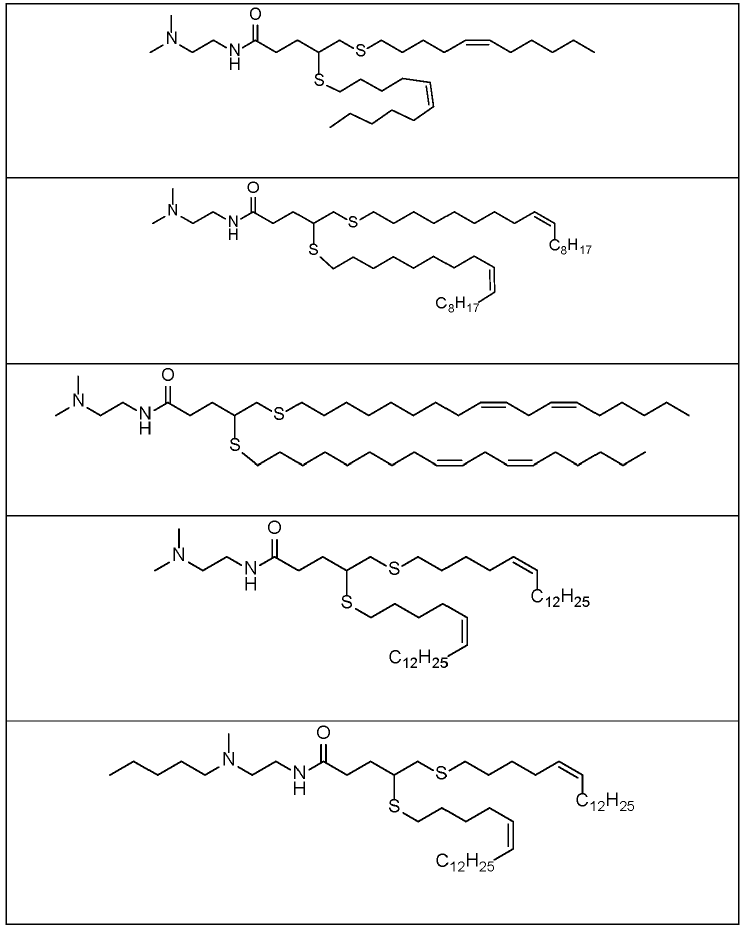

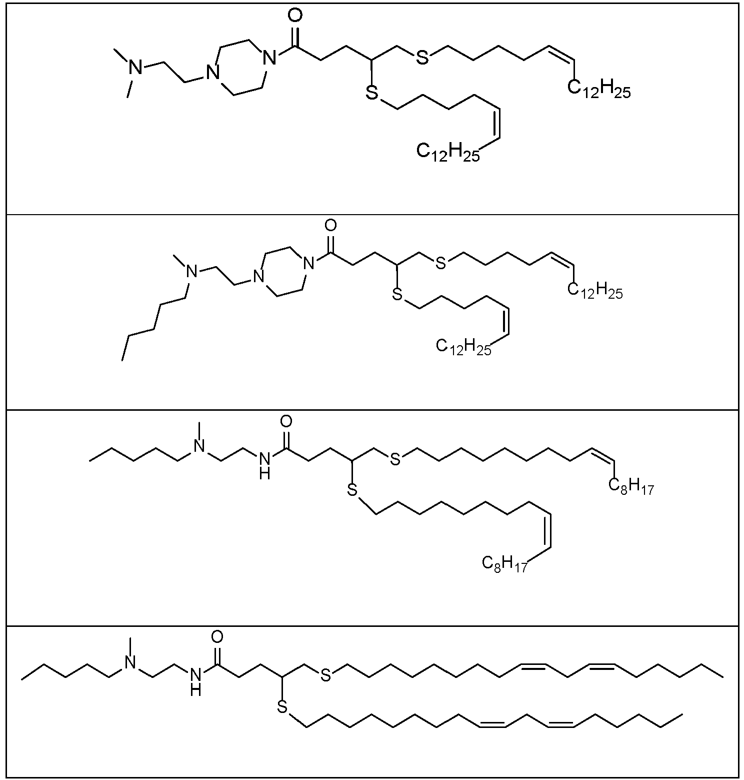

7. A method for synthesizing an amino lipid according to any of claims 1 to 6, comprising the steps of:
a) preparation of an unsaturated alkylthiol by introducing a protective group to an alkyne precursor having one or more triple bonds at the desired locations where the double-bonds are to be located in the amino lipid tail;
b) removal of the protective group;
c) selective reduction of the triple bonds to double bonds, forming an unsaturated intermediate;
d) conversion of the hydroxyl group of the unsaturated intermediate into a thiol;
e) reaction of the intermediate thiol with a methyl ester-derived linker bearing two bromines to form a double-tailed intermediate.
f) amidation of the double-tailed intermediate with an amine to form the final lipid.

8. A lipid particle comprising an amino lipid according to any of claims 1 to 6.

9. A lipid particle according to claim 8, wherein the lipid particle is a lipid nanoparticle (LNP).

10. A lipid particle according to claim 9, further comprising a phospholipid, a sterol and a polymer-lipid conjugate.

11. A lipid particle according to claim 8 to 10, further comprising a payload, the payload preferably being DNA, RNA or protein, most preferably mRNA.

12. The lipid particle according to any of claims 8 to 11, wherein the lipid particle shows an pKa value between about 6.0 and about 7.0, has low toxicity, low hemolytic activity and/or improved fusogenicity.

13. Use of a lipid particle according to any of claims 8 to 12 for delivering a payload into a cell.

14. A pharmaceutical composition comprising an amino lipid according to any of claims 1 to 6 or a lipid particle according to any of claims 8 to 12.

15. A vaccine comprising an amino lipid according to any of claims 1 to 6 or a lipid particle according to any of claims 8 to 12.
